# EUROPÄISCHE PATENTSCHRIFT

(11) **EP 0 609 533 B1**
(45) Veröffentlichungstag und Bekanntmachung des Hinweises auf die Patenterteilung: **20.03.1996**
(21) Anmeldenummer: 93120283.2
(22) Anmeldetag: 16.12.1993
(51) Int. Cl.: C01G 49/00, C01B 13/22, C01G 23/07

(54) **Eisenoxidhaltiges Titandioxidpulver**
Iron oxide containing titanium dioxide
Poudre de bioxyde de titane contenant de l'oxyde de fer

(30) Priorität: 02.02.1993 DE 4302896
(43) Veröffentlichungstag der Anmeldung: 10.08.1994
(73) Patentinhaber: Degussa Aktiengesellschaft, D-60311 Frankfurt (DE)
(72) Erfinder: Hartmann, Werner, Dr., D-64832 Babenhausen (DE); Kerner, Dieter, Dr., D-63450 Hanau (DE)

(56) Entgegenhaltungen:
- EP-A- 0 023 587
- EP-A- 0 241 647
- EP-A- 0 317 875
- DATABASE WPI Derwent Publications Ltd., London, GB; AN 94-022607 & JP-A-5 330 825 (ISHIHARA SANGYO KAISHA)
- PATENT ABSTRACTS OF JAPAN vol. 15, no. 440 (C-0883)11. November 1991 & JP-A-03 186 348 (AGENCY OF IND SCIENCE)
- PATENT ABSTRACTS OF JAPAN vol. 13, no. 260 (C-607)15. Juni 1989 & JP-A-01 061 325 (TAKI CHEM CO LTD)
- DATABASE WPI Derwent Publications Ltd., London, GB; AN 94-022607
- PATENT ABSTRACTS OF JAPAN, Band 15, Nr. 440 (C-0883), 11 November 1991
- PATENT ABSTRACTS OF JAPAN, vol. 13, no. 260 (C-607), 15 Juni 1989

## Beschreibung

Die Erfindung betrifft ein eisenoxidhaltiges Titandioxidpulver, das Verfahren zu seiner Herstellung und Verwendung.

Es ist bekannt, Titandioxid auf pyrogenem Wege, vor allem auf flammenhydrolytischem Wege herzustellen (DE-PS 830 786). Ein derartig hergestelltes Titandioxid kann in Sonnenschutzmittel als UV-absorbierendes Mittel bei gleichzeitiger Transparenz für sichtbares Licht eingesetzt werden. Eine weitere Einsatzmöglichkeit besteht in Lacken und Kunststoffen. Die Herstellung von Mischoxiden auf pyrogenem Wege ist bekannt aus den Dokumenten DE-A 952 891, DE-A 29 31 585, DE-A 29 31 810 und DE-A 36 11 449. Die JP-A-5330825 beschreibt mit Eisenoxid dotiertes Titandioxid, das auf dem Fällungswege hergestellt wird und vorzugsweise mit weiteren Oxiden beschichtet ist. Für besondere Einsatzzwecke, wie z. B. als UV-absorbierendes Agens in Sonnenschutzmitteln, ist eine besonders hohe Asorptionsfähigkeit für UV-Strahlung und eine Transparenz, die mit dem bekannten Titandioxid nicht erreicht wird, notwendig.

Gegenstand der Erfindung ist ein eisenoxidhaltiges Titandioxidpulver, welches dadurch gekennzeichnet ist, daß es aus einem pyrogen, insbesondere flammenhydrolytisch hergestellten Eisenoxid-Titandioxid-Mischoxid mit einer BET-Oberfläche von 10 bis 150 m²/g, welches 0,5 bis 50 Gew.-% Eisenoxid, bezogen auf die Gesamtmenge, als Bestandteile des Mischoxides enthält, besteht.

Es kann hergestellt werden, indem man wasserfreies Eisen-(III)-chlorid verdampft, zusammen mit einem Inertgas, z. B. Stickstoff, in die Mischkammer eines bekannten Brenners überführt, dort mit Wasserstoff, Luft und gasförmigem Titantetrachlorid in einem Verhältnis, das der Zusammensetzung des Eisen-Titan-Mischoxids entspricht, vermischt, das 4-Komponentengemisch in einer Reaktionskammer verbrennt, danach das feste Titandioxid-Mischoxid von den gasförmigen Reaktionsprodukten abtrennt und gegebenenfalls in feuchter Luft von anhaftendem Chlorwasserstoff befreit.

In einer bevorzugten Ausführungsform kann das eisenoxidhaltige Titandioxidpulver (Eisenoxid-Titandioxid-Mischoxid) die folgenden physikalisch-chemischen Parameter aufweisen:

| | |
|---|---|
| Eisenoxid-Gehalt (Gew.-%) | 0,5 - 50 |
| Spezifische Oberfläche (m²/g) | 10 - 150 |
| Primärteilchengröße (nm) | 5 - 100 |
| Stampfdichte (g/l) | 100 - 300 |
| Glühverlust (2 h 1000 °C) (Gew.-%) | 0,5 - 5 |
| Chloridgehalt (Gew.-%) | < 1 |

Es ist sehr feinteilig, sehr homogen und sehr rein. Es weist eine gegenüber dem Stand der Technik bessere Absorption von UV-Licht bei einer weitgehenden Transparenz für sichtbares Licht auf. Es ist gut in dem entsprechenden Medium, zum Beispiel Sonnenschutzmittel, dispergierbar.

Das eisenoxidhaltige Titandioxidpulver kann zur Herstellung von kosmetischen Artikeln, Lacken, Katalysatoren und Photokatalysatoren als UV-Absorber eingesetzt werden.

Ein weiterer Gegenstand der Erfindung sind Hautkosmetika, die eisenoxidhaltiges Titandioxid, vorzugsweise in einer Menge von 0,05 bis 10 Gew.-% enthalten. Das eisenoxidhaltige Titandioxid ist dadurch gekennzeichnet sein, daß es aus einem pyrogen, insbesondere flammenhydrolytisch hergestellten Eisenoxid-Titandioxid-Mischoxid besteht.

### Beispiel 1

FeCl₃ und TiCl₄ werden in zwei getrennten Verdampfern verflüchtigt (Verdampfertemperaturen: FeCl₃ 400 °C, TiCl₄ 200 °C). Die Chloriddämpfe werden mittels Stickstoff in die Mischkammer eines Brenners geleitet. Dort werden sie mit Wasserstoff und getrockneter Luft und/oder Sauerstoff vermischt und in einer Reaktionskammer verbrannt. In der Koagulationsstrecke werden die Reaktionsprodukte auf etwa 100 °C abgekühlt. Die erhaltenen Mischoxide werden anschließend mit einem Filter abgeschieden. Durch eine Behandlung der Mischoxide mit feuchter Luft bei Temperaturen zwischen 500 und 700 °C wird anhaftendes Chlorid entfernt. In der Tabelle 1 sind die Reaktionsbedingungen und die Produkteigenschaften für verschiedene Mischoxide zusammengestellt.

**Tabelle 1**

| Eisen-Titan-Mischoxide | | | | | | | | | |
|---|---|---|---|---|---|---|---|---|---|
| Nr. | TiCl₄ (g/h) | FeCl₃ (g/h) | H₂ (l/h) | Luft (l/h) | Fe₂O₃ (%) | BET (m²/g) | Stampfdichte (g/l) | Glühverlust (%) | Chloridgehalt (%) |
| 1 | 1717 | 14 | 525 | 3079 | 0,9 | 51 | 185 | 2,1 | 0,25 |
| 2 | 1687 | 47 | 525 | 3079 | 3,1 | 47 | 190 | 2,1 | 0,44 |
| 3 | 1613 | 107 | 525 | 3079 | 7,2 | 46 | 185 | 1,7 | 0,36 |
| 4 | 1577 | 161 | 525 | 3079 | 10,7 | 47 | 180 | 1,9 | 0,35 |
| 5 | 1424 | 326 | 525 | 3079 | 21,1 | 49 | 180 | 1,8 | 0,25 |

### Messung der UV-Absorption

Zur Messung der UV-Absorption bzw. Transmission wird das Mischoxid Nr. 2 (3 % Fe₂O₃) in einer Mischung, die 3 Teile Isopropylmyristat und 7 Teile Vaseline enthielt, dispergiert. Der Mischoxid-Anteil beträgt 0,25 Gew.-%. Anschließend wird die Dispersion in einer Schichtdicke von 0,2 mm zwischen zwei Quarz-Platten gebracht. Mit einem Spektrometer UV-201A der Firma Shimazu wird die Transmission gemessen. Als Vergleichssubstanz wird das pyrogene Titandioxid P 25 (BET-Oberfläche 50 m²/g) verwendet und unter den gleichen Bedingungen untersucht.

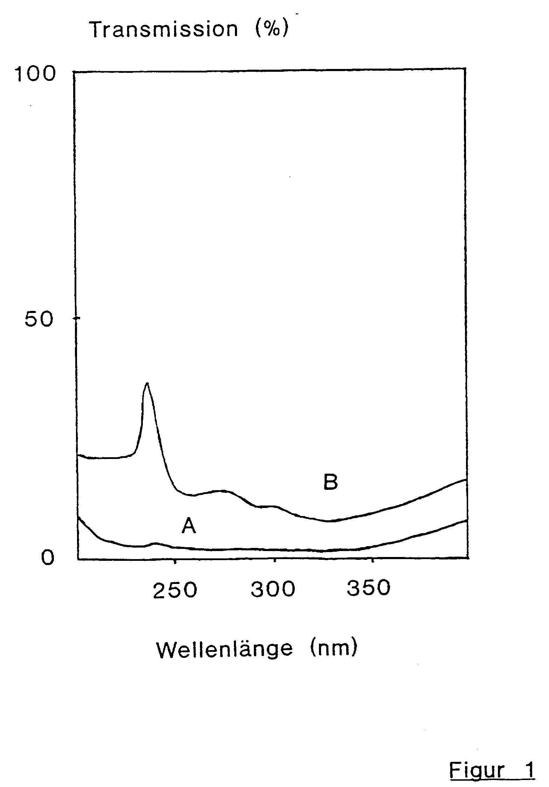

Figur 1 zeigt die Abhängigkeit der Transmission in % von der Wellenlänge im Bereich von 200 bis 400 nm (Kurve A: Mischoxid Nr. 2, Kurve B: Titandioxid P 25). Die Dispersion mit dem Mischoxid zeigt gegenüber der Dispersion, die das reine Titanoxid P 25 enthält, eine deutlich geringere Transmission. Das Mischoxid bietet damit einen ausgezeichneten Schutz gegen UV-Strahlen.

Das Titandioxid P 25 (Hersteller: Degussa AG) ist ein auf pyrogenem Wege aus TiCl₄ hergestelltes Titandioxid. Es weist die folgenden physikalisch-chemischen Kenndaten auf:

| Physikalisch-chemische Daten | | |
|---|---|---|
| | | Titandioxid P 25 |
| BET-Oberfläche | m²/g | 50 ± 15 |
| mittlere Größe der Primärteilchen | Nanometer | 30 |
| Stampfdichte (DIN 53 194) | g/l | ca. 150 |
| Trocknungsverlust * (DIN 53 198, Vefahren A) (2 Stunden bei 105 °C) | % | < 1,5 |
| Glühverlust ** (DIN 52 911) (2 Stunden bei 1000 °C) | % | < 2 |
| pH-Wert (in 4 %iger wäßriger Dispersion) (DIN 53 200) | | 3 - 4 |
| Röntgenstruktur | | überwiegend Anatas |
| isoelektrischer Punkt bei pH-Wert | | 6,6 |
| Dichte | g/cm³ | 3,8 |
| Al₂O₃ ** | % | < 0,3 |
| TiO₂ ** | % | > 97 |
| SiO₂ | % | < 0,2 |
| Fe₂O₃ | % | < 0,01 |
| HCl | % | < 0,3 |

| | | |
|---|---|---|
| * bei Verlassen des Werkes | | |
| ** bezogen auf die 2 Stunden bei 105 °C getrocknete Substanz | | |
| Verpackung: mehrfache Papiersäcke mit Polyäthyleneinlage Aluminiumoxid C: 5 kg netto Titandioxid P 25: 15 kg netto | | |

## Patentansprüche

1. Eisenoxidhaltiges Titandioxidpulver, dadurch gekennzeichnet, daß es aus einem pyrogen hergestellten Eisenoxid-Titandioxid-Mischoxid mit einer BET-Oberfläche von 10 bis 150 m²/g, welches 0,5 bis 50 Gew.-% Eisenoxid, bezogen auf die Gesamtmenge, als Bestandteil des Mischoxides enthält, besteht.

2. Verfahren zur Herstellung des eisenoxidhaltigen Titandioxidpulvers nach Anspruch 1, dadurch gekennzeichnet, daß man wasserfreies Eisen-(III)-chlorid verdampft, zusammen mit einem Inertgas, z. B. Stickstoff, in die Mischkammer eines bekannten Brenners überführt, dort mit Wasserstoff, Luft und gasförmigem Titantetrachlorid in einem Verhältnis, das der Zusammensetzung des Eisenoxid-Titandioxid-Mischoxids entspricht, vermischt, das 4-Komponentengemisch in einer Reaktionskammer verbrennt, danach das feste Eisenoxid-Titandioxid-Mischoxid von den gasförmigen Reaktionsprodukten abtrennt und gegebenenfalls in feuchter Luft von anhaftendem Chlorwasserstoff befreit.

3. Verwendung des pyrogen hergestellten Eisenoxid-Titandioxid-Mischoxides nach Anspruch 1 zur Herstellung von kosmetischen Artikeln, Lacken, Katalysatoren und Photokatalysatoren und als UV-Absorber.

4. Hautkosmetika, enthaltend eisenoxidhaltiges Titandioxid gemäß Ansprüch 1, vorzugsweise in einer Menge von 0,05 bis 10 Gew.-%.

## Claims

1. Titanium dioxide powder containing iron oxide, characterised in that it consists of a pyrogenically produced iron oxide/titanium dioxide mixed oxide with a BET surface area of 10 to 150 m²/g, which contains 0.5 to 50 wt.% of iron oxide, relative to the total quantity, as a constituent of the mixed oxide.

2. Process for the production of the titanium dioxide powder containing iron oxide according to claim 1, characterised in that anhydrous iron(III) chloride is vaporised, transferred together with an inert gas, for example nitrogen, into the mixing chamber of a known burner, is there mixed with hydrogen, air and gaseous titanium tetrachloride in a ratio corresponding to the composition of the iron oxide/titanium dioxide mixed oxide, and the four-component mixture is combusted in a reaction chamber, the solid iron oxide/titanium dioxide mixed oxide is then separated from the gaseous reaction products and any adhering hydrogen chloride is optionally removed in moist air.

3. Use of the pyrogenically produced iron oxide/titanium dioxide mixed oxide according to claim 1 for the production of cosmetic articles, lacquers, catalysts and photocatalysts and as a UV absorber.

4. Skin cosmetic containing titanium dioxide containing iron oxide according to claim 1, preferably in a quantity of 0.05 to 10 wt.%.

## Revendications

1. Poudre de dioxyde de titane contenant de l'oxyde de fer, caractérisée ne ce qu'elle se compose d'un oxyde mixte dioxyde de titane-oxyde de fer produit par pyrogénation avec une surface BET de 10 à 150 m²/g, qui contient de 0,5 à 50 % en poids d'oxyde de fer, rapporté à la quantité totale, en tant que composant de l'oxyde mixte.

2. Procédé de préparation de la poudre de dioxyde de titane contenant du dioxyde de fer selon la revendication 1, caractérisé en ce qu'on vaporise du chlorure de fer (III) anhydre, ensemble avec un gaz inerte, par exemple de l'azote, ou le transfert dans la chambre de carburation d'un brûleur connu, là où le mélange avec de l'hydrogène, de l'air et du tétrachlorure de titane gazeux dans un rapport, qui correspond à la composition de l'oxyde mixte dioxyde de titane-oxyde de fer, on brûle le mélange des 4 composants dans une chambre de réaction, ensuite on sépare l'oxyde mixte solide dioxyde de titane-oxyde de fer des produits de la réaction gazeux et éventuellement on libère dans l'air humide du gaz hydrochlorure adhérent.

3. Utilisation de l'oxyde mixte dioxyde de titane-oxyde de fer selon la revendication 1 pour préparation d'articles cosmétiques, de laques, catalyseurs et photocatalyseurs et comme absorbeur d'UV.

4. Cosmétique, contenant du dioxyde de titane contenant de l'oxyde de fer selon la revendication 1, de préférence en une quantité de 0,05 à 10 % en poids.
